# FASCICULE DE BREVET EUROPEEN

(11) **EP 1 001 751 B1**
(45) Date de publication et mention de la délivrance du brevet: **13.02.2008**
(21) Numéro de dépôt: 98941544.3
(22) Date de dépôt: 05.08.1998
(51) Int. Cl.: A61K 9/48

(54) **COMPOSITIONS VISQUEUSES AQUEUSES, LIMPIDES OU NON, POUR LA FABRICATION DE CAPSULES MOLLES ET DE CAPSULES DURES, ET PROCEDE DE FABRICATION DE FILMS POUR DE TELLES CAPSULES**
WÄSSRIGE, VISKOSE, KLARE ODER NICHT KLARE ZUSAMMENSETZUNGEN ZUR HERSTELLUNG VON WEICHKAPSELN UND HARTKAPSELN, UND HERSTELLUNGSVERFAHREN VON FILMEN FÜR DIESE KAPSELN
AQUEOUS VISCOUS COMPOSITIONS, WHETHER CLEAR OR NOT, FOR MAKING SOFT OR HARD CAPSULES, AND METHOD FOR MAKING FILMS FOR SUCH CAPSULES

(30) Priorité: 08.08.1997 FR 9710190
(43) Date de publication de la demande: 24.05.2000
(73) Titulaire: INTERPHARM DEVELOPMENT, 1020 Remens (CH)
(72) Inventeur: PARIS, Laurence, F-03600 Commentry (FR); VIAUD, Fabrice, F-49280 La Tessoualle (FR)
(74) Mandataire: Thurgood, Alexander John
(86) Numéro de dépôt international: PCT/FR1998/001744
(87) Numéro de publication internationale: WO 1999/007347

(56) Documents cités:
- EP-A- 0 592 130
- EP-A- 0 714 656
- US-A- 5 264 223
- US-A- 5 342 626
- CHEMICAL ABSTRACTS, vol. 126, no. 15, 14 avril 1997 Columbus, Ohio, US; abstract no. 203755, XP002063534 cité dans la demande & JP 09 025228 A (EISAI LTD. CO.,JP) 28 janvier 1997
- CHEMICAL ABSTRACTS, vol. 110, no. 3, 16 janvier 1989 Columbus, Ohio, US; abstract no. 22572, XP002063535 & JP 63 164858 A (UNICOLLOID KK,JP) 8 juillet 1988
- CHEMICAL ABSTRACTS, vol. 103, no. 5, 5 août 1985 Columbus, Ohio, US; abstract no. 36401, XP002063536 cité dans la demande & JP 60 012943 A (AJINOMOTO CO. INC.,JP) 23 janvier 1985
- CHEMICAL ABSTRACTS, vol. 109, no. 18, 31 octobre 1988 Columbus, Ohio, US; abstract no. 156270, XP002063537 cité dans la demande & JP 62 289530 A (EISAI CO. LTD.,JP) 16 décembre 1987
- CHEMICAL ABSTRACTS, vol. 104, no. 25, 23 juin 1986 Columbus, Ohio, US; abstract no. 223917, XP002063538 cité dans la demande & JP 61 010508 A (MITSUBISHI ACETATE CO. LTD.,JP) 18 janvier 1986

## Description

La présente invention concerne le domaine pharmaceutique, cosmétique et diététique et a pour objet la réalisation de films purs de carraghénanes pour la fabrication de gélules ou capsules dures et molles ainsi qu'un procédé d'obtention dudit film et des capsules molles.

Actuellement la tunique des gélules et des capsules est à base de gélatine soit utilisée pure (gélules) ou en association avec différentes substances, glycérine, sorbitol etc... dans le cas des capsules molles.

Or en raison des problèmes potentiels que peut présenter l'origine de la gélatine, en majorité issue d'os de bovins, la maladie dite de la "vache folle", ou BSE (Bovin Spongiform Encéphalite), fait que l'intérêt de pouvoir substituer un tel produit devient crucial.

Un certain nombre de produits ayant des propriétés gélifiantes ou formant des solutions pseudo-colloïdales ont été testés tel que les amidons, les celluloses, et les hydrocolloïdes tels que les alginates, les pectines, les gommes xanthane etc...

Les résultats obtenus ont été peu concluant dans le cas des capsules dures et molles, hormis les dérivés cellulosiques tel que l'hydroxypropylméthylcellulose pour la fabrication des gélules.

Un certain nombre de travaux ont été effectués et ont donné lieu à des brevets. Parmi ceux-ci, les brevets japonais n°09025228, n°62289530, n°61010508 et américain n°5342626 font mention des carraghénanes comme substitut de la gélatine. Or, dans tous les cas, ils se retrouvent toujours en association avec un autre agent gélifiant tels que les manannes, les galactomanes, l'agar, etc... et à des concentrations relativement faibles de l'ordre de 1 à 2 %. Les brevets américain n°5342626 et japonais n°60012943 ne font mention que des carraghénanes Kappa alors qu'il est reconnu que les films obtenus à partir de ce type de carraghénanes présentent un pouvoir de rétraction important : phénomène de synérèse allant à l'encontre de la fabrication de capsules molles ou dures. De plus, ce type de carraghénanes ne permet pas l'obtention d'une solution fortement concentrée fluide à froid comme à chaud.

Selon l'invention, les compositions visqueuses aqueuses ou hydroalcooliques limpides ou non, destinées à la réalisation de films pour capsules molles ou dures (gélules),se caractérisent en ce que l'agent gélifiant unique est constitué par un carragnénane de forme Iota dont la proportion dans le milieu est supérieure à 5% et liée à la présence d'ions alcalins ou alcalino-terreux, l'élasticité du film étant obtenue par l'introduction ou non d'un agent plastifiant, son délitement contrôlé par l'incorporation ou non d'un tensio-actif ou d'un polysaccharide, sa conservation assurée ou non par l'addition de conservateurs, lui permettant ainsi de contenir des solutions huileuses et aqueuses.

L'objet de la présente invention est de réaliser la substitution du film des capsules molles et dures par une tunique à base d'un produit totalement d'origine végétale et très utilisé dans le domaine agro-alimentaire, les carraghénanes employés purs comme seul et unique agent gélifiant de la composition de la tunique avec une concentration supérieure à 5% en solution dans le milieu. Elle est basée sur le fait que les carraghénanes chauffés entre 50° C et 100° C, ont, en refroidissant, la propriété de se gélifier et de donner naissance à des films plus ou moins cassant en fonction du type de carraghénanes utilisés. Le type Iota retenu préférentiellement dans la présente invention ne présente pas le phénomène de synérèse et conduit à des films présentant une certaine élasticité requise pour la fabrication de capsules molles. L'addition d'un certain nombre de substances font que les films obtenus présentent des caractéristiques physiques similaires à ceux de la gélatine tant sur le plan de l'élasticité, de l'épaisseur des films, du temps de désagrégation, de la soudure des films, que sur le plan de leur aspect : films transparents, pouvant être colorés et de découpes variables.

Les carraghénanes sont connus depuis plus de 600 ans dans le domaine médical et en alimentation en particulier pour leur propriété originale qui consistait à gélifier le lait par simple chauffage. Ce sont des polysaccharides, polymères du galactose plus ou moins sulfatés.

Les carraghénanes pouvant être utilisés dans la présente invention sont, en général, extraits à partir de différentes algues : Chondrus crispus, Gigartina stellata, Gigartina acicularis, Gigartina skottsbergii, Gigartina pistillata, Gigartina chamissoi, Iridea, Eucheuma cottoni, Eucheuma spinosum.

Le procédé d'extraction mis en oeuvre conduit à différents types de carraghénanes dont le squelette de base est une chaîne de D-galactoses liés alternativement en α- (1-3) et β - (1-4).

Les différentes qualités sont dues à la quantité et à la position des sulfates et à la présence ou non d'un pont 3, 6 anhydro sur le galactose lié en 1 et 4.

Les différents types de carraghénanes pouvant être utilisés dans la présente invention sont :
- les carraghénanes Iota ;
- les carraghénanes Lambda ;
- les carraghénanes Mu ;
- les carraghénanes Nu ;
les deux derniers étant en plus faible quantité dans la nature.

Les proportions des différents composés varient en fonction de l'espèce de l'algue.

Les différents carraghénanes se différencient par la proportion des groupements soufrés présents sur le squelette de base de la molécule.

Les formes Lambda présentent beaucoup de groupements soufrés comparés aux formes Kappa. Les formes Iota sont intermédiaires.

Les formes Mu et Nu sont en plus faibles quantités et sont considérées comme des impuretés diminuant l'effet gélifiant des formes Iota et Kappa.

La forme Lambda ne présente pas de propriétés gélifiantes mais épaississantes.

Les carraghénanes Iota sont, dans le concept de la présente invention, utilisés seuls sans l'adjonction d'un autre agent gélifiant, contrairement aux carraghénanes utilisés dans l'art antérieur de la fabrication de films pour capsules dures ou molles. De préférence la concentration en carraghénanes sera supérieure à 5% du milieu avec une limite maximum fixée à 80%. Avantageusement le volume de solubilisation des carraghénanes pourra être aussi bien de l'eau qu'un mélange hydroalcoolique dont la proportion en alcool variera entre 0 et 60%.

Le milieu doit être tamponné de manière à éviter une dégradation des carraghénanes dans le temps sous l'effet de la chaleur. En effet, en milieu neutre et sur une période de vingt quatre heures, on observe une diminution de la viscosité du milieu par une hydrolyse progressive des carraghénanes libérant dans le milieu des radicaux acides. cette réaction est bloquée lorsque le milieu est tamponné. La valeur du pH peut varier entre 5 et 12. Différents systèmes tampons peuvent être employés :
- tampon citrates : acide citrique/citrates,
- tampon phosphates : phosphate de sodium ou de potassium, phosphate monosodique/phosphate disodique ou phosphate monopotassique/phosphate dipotassique,
- tampon carbonates : bicarbonate/carbonate,
- tampon phtalates : diphtalate de potassium/acide chlorhydrique,
- tampon borates : acide borique/borate de sodium.

Les agents favorisant la solubilisation des carraghénanes appartiennent à la classe des alcalins et des alcalino-terreux : sodium, calcium, potassium, etc... et sont introduits dans le milieu sous forme :
- de sels des acides chlorhydrique, sulfurique, nitrique, phosphorique, citrique et dérivés ;
- d'hydroxydes.
   La proportion d'ions alcalins et alcalino-terreux pouvant être introduite dans le milieu varie entre 0 et 50 % par rapport au volume final de solution.
   L'élasticité des films est obtenue par l'utilisation de plastifiants qui appartiennent à la classe des polyols : glycérol, sorbitol, maltodextrines, dextrose, mannitol, xylitol, polyoxyéthylène glycol 400 à 6000, glycérides naturels et hemisynthétiques et leurs dérivés, etc...
   La quantité de ces substances introduites dans la solution de carraghénanes est telle que le coefficient d'élasticité du film peut varier de 1 à 5 (1 à 5 fois la longueur initiale). La proportion de ces substances pouvant être introduites dans le milieu varie entre 0 et 30 % par rapport au volume final de solution.
   L'obtention d'un temps de délitement défini du film est contrôlée par l'introduction de tensio-actifs dans le milieu, associés ou non à des substances présentant un pouvoir de désintégration. Les tensio-actifs utilisés dans la présente invention peuvent être
- non ioniques. Ce sont :

- des esters de sorbitane : polysorbates, spans, tweens, etc...
- des acides gras polyéthoxylés : stéarate de PEG 8 au stéarate de PEG 100 ;
- des alcools gras polyéthoxylés : mélange d'éther de monolaurate de PEG ayant de 4 à 23 groupes oxyéthylènes sur la chaîne polyoxyéthylénique, etc...
- des esters de glycol : stéarate de méthylglycol;
- des esters de glycérol : monostéarate de glycérol; etc...
- des esters de PEG ;
- des esters de saccharose ;
- des éthers d'alcool gras et de PEG : Brij ;
- des éthers d'alkyl phénol et de PEG ;
- des tensio- actifs présentant une fonction amide tels que :
   - monoéthanolamide d'acide gras de coprah, d'acide laurique, etc...
   - diéthanolamide d'acide myristique, d'acide laurique, etc...
   - mono-isopropanolamine d'acide laurique.
- ioniques. Ce sont :
   - des dérivés sulfatés : le laurylsulfate de sodium et ses dérivés ;
   - des dérivés sulfonés : dodécylsulfosuccinate de sodium et ses dérivés ;
   - des ammoniums quaternaires : chlorure de cétyltriméthylammonium, laurylpyridinium, distéaryldiméthylammonium, etc...
   - amphotères : bétaïne d'ammonium d'alkyldiméthyle de coprah, dérivés d'amides d'acide gras à structure bétaïnique, acide lauryl-b-iminodipropionique et ses dérivés, acide lauryl-myristyl-b-aminopropionique et ses dérivés, etc...

La quantité de ces substances introduites dans la solution de carraghénanes est telle que le temps de désagrégation peut varier de 3 minutes à 8 heures. Ces quantités peuvent varier de 0 % à 20 % par rapport au volume final de solution. Ces tensio-actifs peuvent être associés à des substances pour améliorer le temps de désagrégation, comme les désintégrants de type amidons de blé, de riz, de maïs, de manioc ayant subi ou non des modifications. les quantités utilisées peuvent varier de 0 à 20% par rapport au volume final de solution.

Des adjuvants de fabrication tels que des conservateurs, des colorants et opacifiants peuvent être introduits dans la solution de carraghénanes. La proportion de conservateurs peut varier de 0 à 10 % par rapport au volume final de solution. Les colorants peuvent être hydrosolubles ou fixés sur une laque d'alumine ou sur tout autre support. Le taux optimum requis se situe entre 0,01 et 5 % pour les colorants et de 1 à 10 % pour les opacifiants par rapport au volume final de solution.

Les solutions ainsi réalisées présentent une viscosité comprise entre 200 et 1.000.000 millipascales. A partir de ces solutions, des films peuvent être réalisés qui présentent une épaisseur au moment de la coulée de l'ordre de 0,5 à 4,0 mm d'épaisseur. Après séchage, les films présentent une épaisseur s'échelonnant entre 0,2 et 2,0 mm d'épaisseur. Leur pouvoir de rétraction après séchage se situe entre 0 et 50 %.

Les films ainsi obtenus peuvent être lubrifiés pour faciliter leur mise en oeuvre au niveau des machines à faire les gélules ou à faire des capsules molles. Les lubrifiants pouvant être utilisés sont :
- des huiles alimentaires classiques (huiles d'arachide, de tournesol, d'olive, etc...),
- des émulsifiants tels que les esters de glycérol et polyoxyéthylène glycol, des triglycérides, des esters de propylène glycol et leurs dérivés.

Ces lubrifiants peuvent être utilisés purs ou dilués avec une dilution de 10 à 75%.

Les films ainsi obtenus pour la fabrication soit des gélules soit des capsules molles peuvent contenir des poudres, des solutions et des suspensions dont les véhicules sont constitués par :
- des huiles : huile d'arachide, de tournesol, d'olive... et de type labrafil ;
- des polyoxyéthyléneglycol : PEG 400, 600 etc...
- des propylène glycols ;
- des émulsionnants : polysorbates, lécithine de soja ;
- des agents de suspension comme des huiles hydrogénées ;
- des solutions aqueuses contenant un ammonium quaternaire.

La présente invention porte également sur le procédé de fabrication des films avec une adaptation pour l'obtention des capsules molles. Ce procédé de fabrication de films à partir de compositions visqueuses selon l'invention consiste :
- à réaliser à chaud une solution tamponnée hydrocolloïdale par dispersion sous vide des carraghénanes seule ou en association dans une solution aqueuse ou hydroalcooliques contenant des ions alcalins ou alcalino-terreux, un plastifiant et un tensio-actif,
- à maintenir cette solution entre 50 et 90°C pendant le stockage,
- et à réaliser les films pour gélules et capsules molles avec une température de scellage maintenue entre 50 et 90°C au moyen de moules préalablement chauffés.

Réalisée à chaud, la fabrication de ces films comporte trois étapes, à savoir :
- la préparation de la solution tamponée des différents constituants : agents de solubilisation, plastifiants, tensio-actifs, conservateurs et colorants ;
- le gonflement des carraghénanes dans la solution de base ;
- et la dispersion si nécessaire, de l'opacifiant.

Un mode de réalisation préférentielle de l'invention consiste, préalablement à l'opération de scellage, à faire subir une déshydratation dudit film pour ramener son taux résiduel d'humidité de l'ordre de 80 à 20%.

Selon une première variante de l'invention, cette opération de déshydratation dudit film est obtenue par chauffage soit par étuvage soit par exposition à des micro-ondes.

Selon une deuxième variante de l'invention, l'opération de déshydratation dudit film est obtenue par congélation.

Les exemples de réalisation ci-après sont des formules de compositions données à titre non limitatif

### Exemple n° 1

| | |
|---|---|
| Carraghénanes | 15 g |
| Chlorure de sodium | 3 g |
| Glycérine | 15 g |
| Eau | 132 g |

### Exemple n° 2

| | |
|---|---|
| Carraghénanes | 15.0 g |
| Hydroxyde de sodium | 1.8 g |
| Glycérine | 7.5 g |
| Eau | 140,7 g |

### Exemple n° 3

| | |
|---|---|
| Carraghénanes | 15.00 g |
| Hydroxyde de sodium | 1.80 g |
| Glycérine | 7.50 g |
| Jaune orangé S | 0.05 g |
| Eau | 140.65 g |

### Exemple 4

| | |
|---|---|
| Carraghénanes | 15.00 g |
| Hydroxyde de sodium | 1.80 g |
| Glycérine | 7.50 g |
| Polysorbate 80 | 1.50 g |
| Eau | 139.20 g |

### Exemple 5

| | |
|---|---|
| Carraghénanes | 15.00 g |
| Chlorure de potassium | 2.00 g |
| Glycérine | 7.50 g |
| Polysorbate 80 | 1.50 g |
| Parahydroxybenzoate de méthyle sodé | 0.12 g |
| Parahydroxybenzoate de propyle sodé | 0.03 g |
| Eau | 139.05 g |

### Exemple 6

| | |
|---|---|
| Carraghénanes | 39.500 g |
| Chlorure de sodium | 1.918 g |
| Glycérine | 6.000 g |
| Polysorbate 80 | 6.000 g |
| Parahydroxybenzoate de méthyle sodé | 0.360 g |
| Parahydroxybenzoate de propyle sodé | 0.090 g |
| Phosphate monosodique | 0.390 g |
| Phosphate disodique | 7.320 g |
| Eau | 300.00 ml |

Dans une cuve de 500 litres à double paroi en inox, pourvue d'un système d'agitation et de vide:
- on introduit 300 kg d'eau, le chlorure de sodium, le phosphate monosodique, le phosphate disodique, le polysorbate 80, la glycérine et les conservateurs,
- on porte la température à 90° C - 100° C,
- on fait le vide et on introduit les carraghénanes sous agitation tout en maintenant la température entre 90° C et 100° C (la vitesse d'agitation est de l'ordre de 1200 à 5000 tr/min et de préférence 2000 tr/min),
- et on maintient l'agitation jusqu'à obtention d'une masse plus ou moins visqueuse. Elle peut être conservée pendant plus de vingt quatre heures à 90°C.

La solution de carraghénanes ainsi obtenue est ensuite transférée vers les machines de fabrication de gélules ou de capsules molles où la température de stockage est maintenue entre 80° C et 90° C.

Dans le cas de capsules molles, le film obtenu subit ensuite une déshydratation préalablement à l'opération de scellage, pour ramener son taux résiduel d'humidité de l'ordre de 80 à 20%. Cette opération de déshydratation est obtenue par chauffage (soit par étuvage soit par exposition à des micro-ondes) ou par congélation à - 4°C pendant trente minutes. Les capsules molles sont obtenues par scellage des films après lubrification selon une adaptation du procédé SCHERER, par chauffage des moules à une température comprise entre 70°C et 100°C.

## Revendications

1. Compositions visqueuses aqueuses ou hydroalcooliques limpides ou non, destinées à la réalisation de films pour capsules molles ou dures (gélules), **CARACTERISEES EN CE QUE** l'agent gélifiant unique est constitué par un carraghénane de forme Iota dont la proportion dans le milieu est supérieure à 5% et liée à la présence d'ions alcalins ou alcalino-terreux, l'élasticité du film étant obtenue par l'introduction ou non d'un agent plastifiant, son délitement contrôlé par l'incorporation ou non d'un tensio-actif ou d'un polysaccharide, sa conservation assurée ou non par l'addition de conservateurs, lui permettant ainsi de contenir des solutions huileuses et aqueuses.

2. Compositions visqueuses aqueuses ou hydroalcooliques selon la revendication 1, **CARACTERISEES EN CE QUE** la proportion d'agent gélifiant s'échelonne de 5 à 80 %.

3. Compositions visqueuses aqueuses ou hydroalcooliques selon la revendication 1, **CARACTERISEES EN CE QUE** la phase aqueuse du milieu de dissolution des carraghénanes est constituée par une solution tampon.

4. Compositions visqueuses aqueuses ou hydroalcooliques selon les revendications 1 et 3, **CARACTERISEES EN CE QUE** le pH de la phase aqueuse tamponnée est compris entre 5 et 12.

5. Compositions visqueuses aqueuses ou hydroalcooliques selon les revendications 1, 3 et 4, **CARACTERISEES EN CE QUE** la phase aqueuse tamponnée est constituée par les couples acide citrique/citrates ou phosphate monosodique/phosphate disodique ou phosphate monopotassique/phosphate dipotassique bicarbonate/carbonate ou diphtalate de potassium/acide chlorydrique ou acide borique/borate de sodium.

6. Compositions visqueuses aqueuses ou hydroalcooliques selon la revendication 1, **CARACTERISEES EN CE QUE** l'agent de solubilisation est un ion alcalin ou alcalino-terreux du type sodium, calcium, potassium, etc...

7. Compositions visqueuses aqueuses ou hydroalcooliques selon la revendication 1, **CARACTERISEES EN CE QUE** la proportion d'ions alcalins ou alcalino-terreux varie de 0 à 50 %.

8. Compositions visqueuses aqueuses ou hydroalcooliques selon les revendications 1 et 7, **CARACTERISEES EN CE QUE** l'ion alcalin ou alcalino-terreux est introduit sous forme d'un hydroxyde ou d'un sel de l'acide chlorhydrique, sulfurique, nitrique, phosphorique, citrique et dérivés.

9. Compositions visqueuses aqueuses ou hydroalcooliques selon la revendication 1, **CARACTERISEES EN CE QUE** l'agent plastifiant est un polyol du type sorbitol, glycérine, mannitol, xylitol, lactitol, polyoxyéthylène glycols, propylène glycol et leurs dérivés et des glycérides hemisynthétiques et leurs dérivés.

10. Compositions visqueuses aqueuses ou hydroalcooliques selon la revendication 1, **CARACTERISEES EN CE QUE** la proportion d'agent plastifiant varie de 0 à 30 %.

11. Compositions visqueuses aqueuses ou hydroalcooliques selon la revendication 1, **CARACTERISEES EN CE QUE** le tensio-actif assurant le contrôle du délitement du film appartient aux classes des tensio-actifs ioniques, non ioniques et amphotères.

12. Compositions visqueuses aqueuses ou hydroalcooliques selon les revendications 1 et 10, **CARACTERISEES EN CE QUE** le tensio-actif assurant le contrôle du délitement du film appartient aux classes des tensio-actifs ioniques, non ioniques et amphotères et est associé à des désintégrants de type amidon.

13. Compositions visqueuses aqueuses ou hydroalcooliques selon les revendications 1 et 10, **CARACTERISEES EN CE QUE** la teneur en tensio-actif varie de 0 à 20 %.

14. Compositions visqueuses aqueuses ou hydroalcooliques selon les revendications 1 et 12, **CARACTERISEES EN CE QUE** la teneur en amidons et leurs dérivés est comprise entre 0 à 20 %.

15. Compositions visqueuses aqueuses ou hydroalcooliques selon les revendications 1 à 10, **CARACTERISEES EN CE QUE** des adjuvants de conservation et de coloration sont introduits.

16. Compositions visqueuses aqueuses ou hydroalcooliques selon la revendication 15, **CARACTERISEES EN CE QUE** la teneur en conservateurs s'échelonne de 0 à 10 % du volume final.

17. Compositions visqueuses aqueuses ou hydroalcooliques selon la revendication 15, **CARACTERISEES EN CE QUE** la teneur en colorants s'échelonne de 0,01 à 5 % du volume final.

18. Compositions visqueuses aqueuses ou hydroalcooliques selon l'une quelconque des revendications 1 à 17, **CARACTERISEES EN CE QUE** les films obtenus à partir desdites compositions visqueuses aqueuses sont lubrifiés par des huiles alimentaires classiques ou des esters de glycérol et polyoxyéthylène glycol, des triglycérides, des esters de propylène glycol et leurs dérivés ou des solutions diluées de ces différents produits.

19. Compositions visqueuses aqueuses ou hydroalcooliques selon l'une quelconque des revendications 1 à 17, **CARACTERISEES EN CE QUE** les liquides susceptibles être contenus dans les capsules sont des solutions aqueuse et huileuse.

20. Procédé de fabrication de films à partir de compositions visqueuses selon l'ensemble des revendications 1 à 19, **CARACTERISE EN CE QU'**il consiste :
- à réaliser à chaud une solution tamponnée hydrocolloïdale par dispersion sous vide des carraghénanes seules ou en association dans une solution aqueuse ou hydroalcooliques contenant des ions alcalins ou alcalino-terreux, un plastifiant et un tensio-actif,
- à maintenir cette solution entre 50 et 90°C pendant le stockage,
- et à réaliser les films pour gélules et capsules molles avec une température de scellage maintenue entre 50 et 90°C au moyen de moules préalablement chauffés.

21. Procédé de fabrication selon la revendication 20, **CARACTERISE EN CE QU'**il consiste, préalablement à l'opération de scellage, à faire subir une déshydratation dudit film pour ramener son taux résiduel d'humidité de l'ordre de 80 à 20%.

22. Procédé de fabrication selon la revendication 21, **CARACTERISE EN CE QUE** l'opération de déshydratation dudit film est obtenue par chauffage soit par étuvage soit par exposition à des micro-ondes.

23. Procédé de fabrication selon la revendication 21, **CARACTERISE EN CE QUE** l'opération de déshydratation dudit film est obtenue par congélation.

## Claims

1. Aqueous viscous compositions, whether clear polyhydric alcohols or not, for the making of soft or hard capsules (gelled capsules), wherein the single gelling agent is constitued by an Iota carrageenaan whereof the concentration in the medium is higher than 5% and linked to the presence of alkaline or alkaline earths ions, the elasticity of the film being obtained by the introduction or not of a plastified agent, its disssolution controlled by the blending in or not of a surfactant or a polysaccharide, its preservation being ensured or not by the addition of preservatives, enabling it to contain aqueous and oily solutions.

2. Aqueous viscous compositions, or polyhydric alcohols according to claim 1, wherein the proportion of gelling agents ranges from 5 to 80%.

3. Aqueous viscous compositions, or polyhydric alcohols according to claim 1, wherein the aqueous stage in the medium of dissolution of the carrageenans is constitued by a buffer solution.

4. Aqueous viscous compositions, or polyhydric alcohols according to claims 1 and 3, wherein the PH of the buffered aqueous stage is comprised between 5 and 12.

5. Aqueous viscous compositions, or polyhydric alcohols according to claims 1, 3 and 4, wherein the buffered aqueous stage is constitued by the citric acid/citrate couples or monosodium dihydrogen phosphate/disodium monohydrogen phosphate or monopotassium dihydrogen phosphate/dipotassium monohydrogen phosphate or bicarbonate/carbonate or potassium diphtalate/hydrochloric acid or boric acid/sodium borate.

6. Aqueous viscous compositions, or polyhydric alcohols according to claim 1, wherein the dissolution agent is an alkaline or an alkaline earth ion of the sodium, calcium, potassium type, etc.

7. Aqueous viscous compositions, or polyhydric alcohols according to claim 1, wherein the proportion of alkaline ions or alkaline earths varies between 0 to 50% of the composition.

8. Aqueous viscous compositions or polyhydric alcohols according to claims 1 and 7, wherein the alkaline or alkaline earth ion is introduced in the shape of a hydroxyde or a salt of the hydrochloric acid, sulfuric acid, phosphoric acid, citric acid and their derivatives.

9. Aqueous viscous compositions, or polyhydric alcohols according to claim 1, wherein the plasticizing agent is a polyoxyl of the type : sorbitol, glycerine, mannitol, xylitol, lactitol, polyoxyethylene glycols, propylene glycols and their derivatives and semisynthetic glycerides and their derivatives.

10. Aqueous viscous compositions, or polyhydric alcohols according to claim 1, wherein the proportion of plasticizing agents varies from 0 to 30% of the composition.

11. Aqueous viscous compositions, or polyhydric alcohols according to claim 1, wherein the surfactant ensuring the dissolution control of the film belongs to the class of ionic surfactants, non ionic surfactants and amphoteric surfactants.

12. Aqueous viscous compositions, or polyhydric alcohols according to claims 1 and 10, wherein the surfactant ensuring the dissolution control of the film belongs to the class of ionic surfactants, non ionics surfactants and amphoterics surfactants and is combined with disintegrators of the starch type.

13. Aqueous viscous compositions, or polyhydric alcohols according to claims 1 and 10, wherein the concentration of surfactant varies from 0 to 20% of the composition.

14. Aqueous viscous compositions, or polyhydric alcohols according to claims 1 and 12, wherein the concentration of starch and their derivatives is comprised between 0 and 20% of the composition.

15. Aqueous viscous compositions, or polyhydric alcohols according to claims 1 to 10, wherein the additives for preservation and colouring are introduced.

16. Aqueous viscous compositions, or polyhydric alcohols according to claim 15, wherein the concentration of the preservative ranges from 0 to 10% of the final volume of the composition.

17. Aqueous viscous compositions, or polyhydric alcohols according to claim 15, wherein the content of colourings ranges from 0,01 to 5% of the final volume.

18. Aqueous viscous compositions, or polyhydric alcohols according to any of the claims 1 to 17, wherein the films obtained from the said aqueous viscous compositions are lubricated by oils or esters of glycerol and esters polyoxyethylene glycol, triglycerids, esters of propylene glycol and their derivatives or diluted solutions of these different products.

19. Aqueous viscous compositions, or polyhydric alcohols according to any of the claims 1 to 17, wherein the liquids susceptible of being contained in the capsules are aqueous and oily solutions.

20. A process for forming films from viscous compositions according to the whole of the claims 1 to 19, wherein it consists of :
- making in hot work a hydrocolloidal buffered solution by dispersion under vacuum of the carrageenans on their own or in association in an aqueous solution or a polyhydric alcohols one containing alkaline or alkaline earths ions, a plasticizer and a surfactant,
- maintaining this solution between 50 and 90°C during storage,
- and forming films for hard and soft capsules with a sealing temperature maintained between 50°C and 90°C by means of preheated dies forming.

21. A process for forming films according to claim 20, wherein it consists of, before the sealing operation, treating the said film by dehydrating it to bring its residual moisture level to about 80% to 20%.

22. A process for forming films according to claim 21, wherein the dehydration operation of the said film is obtained by heating either by oven drying or by exposure to micro-waves.

23. A process for forming films according to claim 21, wherein the dehydration operation of the said film is obtained by freezing.

## Patentansprüche

1. Viskose, wässrige oder hydroalkoholische, klare oder nicht klare Zusammensetzungen, die zur Herstellung von Filmen für Weichkapseln oder Hartkapseln (Gelatinekapseln) bestimmt sind, **dadurch gekennzeichnet, dass** das einzige Geliermittel aus einem Carrageenan der Form Jota gebildet ist, dessen Anteil im Medium über 5% beträgt und mit der Gegenwart von Alkali- oder Erdalkaliionen verbunden ist, wobei die Elastizität des Films durch die Einführung oder Nichteinführung eines Plastifizierungsmittels erreicht wird, seine Auflösung durch den Einbau oder Nichteinbau eines grenzflächenaktiven Stoffs oder eines Polysaccharids kontrolliert wird und seine Konservierung durch den Zusatz von Konservierungsmitteln gesichert wird oder nicht, wobei es ihm auf diese Weise möglich ist, ölige oder wässrige Lösungen zu enthalten.

2. Viskose, wässrige oder hydroalkoholische Zusammensetzungen nach Anspruch 1, **dadurch gekennzeichnet, dass** der Anteil des Geliermittels zwischen 5 und 80% beträgt.

3. Viskose, wässrige oder hydroalkoholische Zusammensetzungen nach Anspruch 1, **dadurch gekennzeichnet, dass** die wässrige Phase des Mediums zur Lösung der Carrageenane durch eine Pufferlösung gebildet ist.

4. Viskose, wässrige oder hydroalkoholische Zusammensetzungen nach den Ansprüchen 1 und 3, **dadurch gekennzeichnet, dass** der pH-Wert der gepufferten wässrigen Phase zwischen 5 und 12 beträgt.

5. Viskose, wässrige oder hydroalkoholische Zusammensetzungen nach den Ansprüchen 1, 3 und 4, **dadurch gekennzeichnet, dass** die gepufferte wässrige Phase durch die Paare Zitronensäure/Citrate oder Mononatriumphosphat/Dinatriumphosphat oder Monokaliumphosphat/Dikaliumphosphat oder Bicarbonat/Carbonat oder Kaliumdiphthalat/Salzsäure oder Borsäure/Natriumborat gebildet ist.

6. Viskose, wässrige oder hydroalkoholische Zusammensetzungen nach Anspruch 1, **dadurch gekennzeichnet, dass** das Solubilisierungsmittel ein Alkali- oder Erdalkaliion vom Typ Natrium, Calcium, Kalium usw. ist.

7. Viskose, wässrige oder hydroalkoholische Zusammensetzungen nach Anspruch 1, **dadurch gekennzeichnet, dass** der Anteil der Alkali- oder Erdalkaliionen von 0 bis 50% variiert.

8. Viskose, wässrige oder hydroalkoholische Zusammensetzungen nach den Ansprüchen 1 und 7, **dadurch gekennzeichnet, dass** das Alkali- oder Erdalkaliion in Form eines Hydroxids oder eines Salzes der Salzsäure, Schwefelsäure, Salpetersäure, Phosphorsäure, Zitronensäure und Derivaten eingeführt wird.

9. Viskose, wässrige oder hydroalkoholische Zusammensetzungen nach Anspruch 1, **dadurch gekennzeichnet, dass** das Plastifizierungsmittel ein Polyol vom Typ Sorbitol, Glycerin, Mannitol, Xylitol, Lactitol, Polyoxyethylenglycol, Propylenglycol und deren Derivate sowie der halbsynthetischen Glyceride und deren Derivate ist.

10. Viskose, wässrige oder hydroalkoholische Zusammensetzungen nach Anspruch 1, **dadurch gekennzeichnet, dass** der Anteil des Plastifizierungsmittels von 0 bis 30% variiert.

11. Viskose, wässrige oder hydroalkoholische Zusammensetzungen nach Anspruch 1, **dadurch gekennzeichnet, dass** der grenzflächenaktive Stoff, der die Kontrolle der Auflösung des Films sichert, zu den Klassen der ionischen, nichtionischen und amphoteren grenzflächenaktiven Stoffe gehört.

12. Viskose, wässrige oder hydroalkoholische Zusammensetzungen nach den Ansprüchen 1 und 10, **dadurch gekennzeichnet, dass** der grenzflächenaktive Stoff, der die Kontrolle der Auflösung des Films sichert, zu den Klassen der ionischen, nichtionischen und amphoteren grenzflächenaktiven Stoffe gehört und mit Zersetzungsmitteln vom Stärketyp kombiniert wird.

13. Viskose, wässrige oder hydroalkoholische Zusammensetzungen nach den Ansprüchen 1 und 10, **dadurch gekennzeichnet, dass** der Gehalt an grenzflächenaktivem Stoff von 0 bis 20% variiert.

14. Viskose, wässrige oder hydroalkoholische Zusammensetzungen nach den Ansprüchen 1 und 12, **dadurch gekennzeichnet, dass** der Gehalt an Stärken und ihren Derivaten von 0 bis 20% variiert.

15. Viskose, wässrige oder hydroalkoholische Zusammensetzungen nach den Ansprüchen 1 bis 10, **dadurch gekennzeichnet, dass** Zusatzsstoffe zur Konservierung und Färbung eingeführt werden.

16. Viskose, wässrige oder hydroalkoholische Zusammensetzungen nach Anspruch 15, **dadurch gekennzeichnet, dass** der Gehalt an Konservierungsstoffen zwischen 0 und 10% des endgültigen Volumens beträgt.

17. Viskose, wässrige oder hydroalkoholische Zusammensetzungen nach Anspruch 15, **dadurch gekennzeichnet, dass** der Gehalt an Farbstoffen zwischen 0,01 und 5% des endgültigen Volumens beträgt.

18. Viskose, wässrige oder hydroalkoholische Zusammensetzungen nach einem der Ansprüche 1 bis 17, **dadurch gekennzeichnet, dass** die Filme, die ausgehend von den genannten viskosen wässrigen Zusammensetzungen erzielt werden, durch herkömmliche Speiseöle oder Glycerolester und Polyoxyethylenglycol, Triglyceride, Propylenglycolester und ihre Derivate oder verdünnte Lösungen dieser verschiedenen Produkte geschmiert werden.

19. Viskose, wässrige oder hydroalkoholische Zusammensetzungen nach einem der Ansprüche 1 bis 17, **dadurch gekennzeichnet, dass** die Flüssigkeiten, die in den Kapseln enthalten sein können, wässrige und ölige Lösungen sind.

20. Verfahren zur Herstellung von Filmen ausgehend von viskosen Zusammensetzungen nach der Gesamtheit der Ansprüche 1 bis 19, **dadurch gekennzeichnet, dass** es darin besteht,
- eine hydrokolloidale, gepufferte Lösung durch Dispersion der Carrageenane im Vakuum, allein oder in Kombination, in einer wässrigen oder hydroalkoholischen Lösung, die Alkali- oder Erdalkaliionen, ein Plastifizierungsmittel und einen grenzflächenaktiven Stoff enthält, unter Wärme herzustellen;
- diese Lösung während der Lagerung auf 50 bis 90°C zu halten;
- und die Filme für Gelatinekapseln und Weichkapseln mit einer Siegeltemperatur zwischen 50 und 90°C mit Hilfe von vorgewärmten Formen herzustellen.

21. Herstellungsverfahren nach Anspruch 20, **dadurch gekennzeichnet, dass** es darin besteht, vor dem Versiegelungsvorgang den Film einem Entwässerungsvorgang zu unterziehen, um seine Restfeuchtigkeit auf einen Wert in der Größenordnung von 80 bis 20% zu bringen.

22. Herstellungsverfahren nach Anspruch 21, **dadurch gekennzeichnet, dass** der Vorgang der Entwässerung des Films durch Erhitzen erfolgt, und zwar durch Ofentrocknung oder durch Bestrahlung mit Mikrowellen.

23. Herstellungsverfahren nach Anspruch 21, **dadurch gekennzeichnet, dass** der Vorgang der Entwässerung des Films durch Gefrieren erfolgt.
